# EUROPEAN PATENT SPECIFICATION

(11) **EP 4 731 149 B1**
(45) Date of publication and mention of the grant of the patent: **30.09.2026**
(21) Application number: 24798273.9
(22) Date of filing: 17.10.2024
(51) Int. Cl.: A61F 13/05

(54) **SEALING STRIP**
DICHTBAND
BANDE D'ÉTANCHÉITÉ

(30) Priority: 19.10.2023 US 202363591608 P; 07.12.2023 GB 202318727
(43) Date of publication of application: 29.04.2026
(73) Proprietor: ConvaTec Limited, Deeside, Flintshire CH5 2NU (GB)
(72) Inventor: BROWN, Natalie, Deeside Flintshire CH5 2NU (GB); DAVIES, Liam, Deeside Flintshire CH5 2NU (GB); GASKELL, Beth, Deeside Flintshire CH5 2NU (GB); EADE, Annchalee, Deeside Flintshire CH5 2NU (GB); KESTEVEN, Donna, Deeside Flintshire CH5 2NU (GB); BALLAMY, Lucy, Deeside Flintshire CH5 2NU (GB)
(74) Representative: Wilson Gunn
(86) International application number: PCT/GB2024/052659
(87) International publication number: WO 2025/083405

(56) References cited:
- US-A1- 2016 367 737
- US-B2- 10 231 878
- US-B2- 10 493 184
- US-B2- 10 842 707

## Description

### Technical Field of the Invention

The present invention relates to a sealing strip for a negative pressure wound dressing and a negative pressure wound therapy kit comprising at least one sealing strip.

### Background to the Invention

Negative pressure wound therapy (NPWT) is a wound dressing system wherein a sub-atmospheric pressure is applied intermittently or continuously to a wound site. NPWT can be used on acute or chronic wounds and can be advantageous for a patient because it can lead to quicker healing and reduced likelihood of infection.

Typically negative pressure wound therapy dressings are secured to a patient's skin by an adhesive surface on the dressing. Additional sealing strips can then be applied to the edges of the wound dressing to ensure that a tight seal is formed such that the sub-atmospheric pressure can be successfully applied to the wound without significant air leakage. Frequent handling of part or all of the NPWT dressing could introduce bacteria into the wound thereby leading to infection; therefore, to avoid having to frequently replace the sealing strip or the entire NPWT dressing it would be advantageous to provide a sealing strip with an increased wear time.

Improved wear time can be achieved through high adhesion, but high adhesion could also result in the sealing strip being painful to remove when it does need to be removed or replaced, for example to examine the wound or change the dressing to a clean dressing. It would therefore be advantageous to provide a sealing strip with improved adhesion leading to increased wear time which, when required, doesn't require excess force to remove or cause pain to the patient.

Sealing strips require a high moisture vapour transfer rate (MVTR) so that moisture can be removed from the skin and does not become trapped between the sealing strip and the skin. Trapped moisture can negatively impact the adhesion of the sealing strip and cause maceration of the peri-wound skin. Improved MVTR can be achieved with thinner sealing strips however thinner sealing strips can be more difficult to apply. If the sealing strip is difficult to apply it may crease or fold on application and therefore would not have as long a wear time and there may be increased likelihood of the strips detaching from the wound dressing and skin during use. It would therefore be advantageous to provide a sealing strip which has both an improved MVTR and improved handling and therefore improved wear time.

US10231878 discloses a negative pressure wound therapy apparatus comprising a wound contact layer with a silicone adhesive to promote scar-free healing at a tissue site where negative pressure is applied.

It is an aim of embodiments of the invention to overcome one or more problems of the prior art, whether expressly disclosed herein or not.

### Summary of the Invention

According to a first aspect of the invention there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm.

The sealing strip of the invention may be advantageous because the adhesive layer having a weight of no more than 33 gsm and the carrier layer having a weight of between 20 gsm and 35 gsm results in a good moisture vapour transfer rate because the moisture vapour has a shorter distance to travel. The sealing strip may also be advantageous because it is easy to apply to the skin without creasing or folding thereby leading to a longer wear time.

The adhesive layer may have a weight of no more than 32 gsm, 31 gsm, 30 gsm, 29 gsm, 28 gsm, 27 gsm, 26 gsm, 25 gsm, 24 gsm, 23 gsm, 22 gsm, 20 gsm, 18 gsm, 16 gsm or no more than 15 gsm. This embodiment may be advantageous because the low weight, thin adhesive layer results in an improved moisture vapour transfer rate so that moisture is successfully removed from the skin and does not become trapped between the skin and the adhesive layer which can have a negative impact on the adhesion of the sealing strip.

The acrylic adhesive may comprise no more than 20 wt.% solvent. The acrylic adhesive may comprise no more than 18 wt.%, 15 wt.%, 12 wt.%, 10 wt.%, 8 wt.% or no more than 5 wt.% solvent. In some embodiments the adhesive layer is an essentially solvent free adhesive layer. This embodiment may be advantageous because it is more environmentally friendly due to the low concentration of volatile organic compounds.

The acrylic adhesive may be chosen from ethyl acrylate, butyl acrylate and methyl acrylate or any combination thereof. The acrylic adhesive may be made from an acrylate selected from the group consisting of: ethyl acrylate, butyl acrylate and methyl acrylate or any combination thereof.

The carrier layer may have a weight of between 25 gsm and 35 gsm, 20 gsm and 30 gsm, 22 gsm and 30 gsm, or between 24 gsm and 30 gsm, or between 25 gsm and 29 gsm, or between 25 gsm and 28 gsm. This embodiment may be advantageous because it leads to an improved MVTR.

In some embodiments the adhesive layer has a weight of no more than 33 gsm, and the carrier layer has a weight of between 25 gsm and 35 gsm. In some embodiments the adhesive layer has a weight of no more than 30 gsm, and the carrier layer has a weight of between 25 gsm and 35 gsm. In some embodiments the adhesive layer has a weight of no more than 30 gsm, and the carrier layer has a weight of between 25 gsm and 28 gsm. In some embodiments the adhesive layer has a weight of no more than 29 gsm, and the carrier layer has a weight of between 22 gsm and 30 gsm. In some embodiments the adhesive layer has a weight of no more than 29 gsm, and the carrier layer has a weight of between 24 gsm and 30 gsm. In some embodiments the adhesive layer has a weight of no more than 29 gsm, and the carrier layer has a weight of between 25 gsm and 30 gsm. In some embodiments the adhesive layer has a weight of no more than 27 gsm, and the carrier layer has a weight of between 25 gsm and 29 gsm. In some embodiments the adhesive layer has a weight of no more than 27 gsm, and the carrier layer has a weight of between 25 gsm and 30 gsm. In some embodiments the adhesive layer has a weight of no more than 27 gsm, and the carrier layer has a weight of between 25 gsm and 28 gsm. In some embodiments the adhesive layer has a weight of no more than 25 gsm, and the carrier layer has a weight of between 20 gsm and 30 gsm. In some embodiments the adhesive layer has a weight of no more than 25 gsm, and the carrier layer has a weight of between 25 gsm and 30 gsm. In some embodiments the adhesive layer has a weight of no more than 25 gsm, and the carrier layer has a weight of between 25 gsm and 28 gsm. This embodiment is advantageous because it results in a sealing strip with improved MVTR and reduced folding or wrinkling in use which in turn results in a longer wear time.

The thickness of the carrier layer and the adhesive layer may be no more than 0.058 mm. The thickness of the carrier layer and the adhesive layer may be no more than 0.057, 0.056, 0.055, 0.054, 0.053, 0.052 or preferably no more than 0.051 mm. The reduced thickness of the carrier layer and the adhesive layer may be advantageous because it may result in a sealing strip with improved MVTR. The reduced thickness of the carrier layer and the adhesive layer in combination with the release liners may result in a sealing strip with reduced folding or wrinkling in use which in turn results in a longer wear time.

In some embodiments the carrier layer comprises a polymer. The polymer may be chosen from polylactic acid, polycaprolactone, polyethylene (LDPE, HDPE), polypropylene, polyester, polyamide, ethylene vinyl acetate and polyurethane or any combination thereof. The polymer may be selected from a group consisting of: polylactic acid, polycaprolactone, polyethylene (LDPE, HDPE), polypropylene, polyester, polyamide, ethylene vinyl acetate and polyurethane, or any combination thereof. In preferred embodiments the carrier comprises polyurethane. The carrier may comprise a polyurethane film with a weight of between 20 gsm and 35 gsm, 25 gsm and 35 gsm, 20 gsm and 30 gsm or between 22 gsm and 28 gsm. Embodiments wherein the carrier comprises polyurethane may be advantageous because polyurethane is biologically safe, able to be sterilised and flexible.

The carrier layer may be transparent or translucent. This may be advantageous because it may allow the user to accurately place the sealing strip at the desired site.

In some embodiments the adhesive layer covers at least 50 % of the front surface of the carrier layer. The adhesive layer may cover at least 55 %, 60 %, 65 %, 70 %, 75 %, 80 %, 85 %, 90 %, 95 %, 96 %, 97 %, 98 % or at least 99 % of the front surface of the carrier layer. In some embodiments the adhesive layer covers substantially the entire front surface of the carrier layer or the entire front surface of the carrier layer.

In some embodiments at least two release liners are detachably adhered to the adhesive layer. In some embodiments three release liners are detachably adhered to the adhesive layer. The release liner(s) may be partially overlapping. The release liner(s) may be non-overlapping.

The release liner(s) may comprise paper release liner(s). The release liner(s) may comprise a polymer film. The polymer of the polymer film may be chosen from polypropylene, polyethylene, polyurethane and polyester or any combination thereof. The polymer of the polymer film may be selected from the group consisting of: polypropylene, polyethylene, polyurethane and polyester or any combination thereof. The release liner(s) may comprise a polymer coated paper release liner. The release liner(s) may comprise a siliconized paper release liner (also known as a silicone coated release liner).

The release liner(s) may have a weight of between 50 gsm and 80 gsm. The release liner(s) may have a weight of between 55 gsm and 80 gsm, or between 55 gsm and 75 gsm, or between 55 gsm and 70 gsm, or between 55 gsm and 65 gsm or a weight of between 60 gsm and 65 gsm.

In some embodiments the adhesive layer has a weight of no more than 33 gsm, and the carrier layer has a weight of between 25 gsm and 35 gsm. In some embodiments the adhesive layer has a weight of no more than 30 gsm, and the carrier layer has a weight of between 25 gsm and 35 gsm, and the release liner has a weight of between 50 gsm and 80 gsm. In some embodiments the adhesive layer has a weight of no more than 30 gsm, and the carrier layer has a weight of between 25 gsm and 28 gsm, and the release liner has a weight of between 50 gsm and 80 gsm. In some embodiments the adhesive layer has a weight of no more than 33 gsm, and the carrier layer has a weight of between 25 gsm and 35 gsm. In some embodiments the adhesive layer has a weight of no more than 30 gsm, and the carrier layer has a weight of between 25 gsm and 35 gsm, and the release liner has a weight of between 55 gsm and 70 gsm. In some embodiments the adhesive layer has a weight of no more than 30 gsm, and the carrier layer has a weight of between 25 gsm and 28 gsm, and the release liner has a weight of between 55 gsm and 70 gsm. In some embodiments the adhesive layer has a weight of no more than 29 gsm, the carrier layer has a weight of between 22 gsm and 30 gsm and the release liner has a weight of between 50 gsm and 80 gsm. In some embodiments the adhesive layer has a weight of no more than 29 gsm, the carrier layer has a weight of between 29 gsm and 30 gsm and the release liner has a weight of between 55 gsm and 75 gsm. In some embodiments the adhesive layer has a weight of no more than 27 gsm, the carrier layer has a weight of between 25 gsm and 30 gsm and the release liner has a weight of between 55 gsm and 60 gsm. In some embodiments the adhesive layer has a weight of no more than 27 gsm, the carrier layer has a weight of between 24 gsm and 30 gsm and the release liner has a weight of between 50 gsm and 80 gsm. In some embodiments the adhesive layer has a weight of no more than 25 gsm, the carrier layer has a weight of between 20 gsm and 30 gsm and the release liner has a weight of between 50 gsm and 80 gsm. In some embodiments the adhesive layer has a weight of no more than 25 gsm, the carrier layer has a weight of between 20 gsm and 30 gsm and the release liner has a weight of between 55 gsm and 65 gsm. In some embodiments the adhesive layer has a weight of no more than 25 gsm, the carrier layer has a weight of between 25 gsm and 30 gsm and the release liner has a weight of between 50 gsm and 80 gsm. In some embodiments the adhesive layer has a weight of no more than 25 gsm, the carrier layer has a weight of between 25 gsm and 30 gsm and the release liner has a weight of between 55 gsm and 65 gsm. In some embodiments the adhesive layer has a weight of no more than 25 gsm, the carrier layer has a weight of between 25 gsm and 28 gsm and the release liner has a weight of between 50 gsm and 80 gsm. In some embodiments the adhesive layer has a weight of no more than 25 gsm, the carrier layer has a weight of between 25 gsm and 28 gsm and the release liner has a weight of between 55 gsm and 65 gsm. This embodiment is advantageous because it results in a sealing strip with improved MVTR and reduced folding or wrinkling in use which in turn results in a longer wear time. A portion of the or each release liner may comprise a handling tab formed from a portion of the release liner which is not connected to the adhesive layer. In some embodiments at least one of the release liners may comprise two handling tabs each formed from a portion of the release liner which is not connected to the adhesive layer.

The sealing strip may comprise at least one protective liner covering at least a portion of the rear surface of the carrier layer. The protective liner may cover at least 50 % of the rear surface of the carrier layer. The protective liner may cover at least 55 %, 60 %, 65 %, 70 %, 75 %, 80 %, 85 %, 90 %, 95 %, 96 %, 97 %, 98 % or at least 99 % of the rear surface of the carrier layer. In some embodiments the protective liner covers substantially the entire rear face or the entire rear face of the carrier layer. The protective liner may extend beyond at least one edge of the carrier layer, preferably at least one lateral edge. This embodiment may be advantageous because in use it allows a clinician or a patient to easily remove the protective liner without peeling or disrupting the edge of the sealing strip; peeling or disrupting the edge of the sealing strip may lead to increased lifting and therefore a shorter wear time.

The protective liner may comprise a paper protective liner. The protective liner may comprise a polymer film. The polymer may be chosen from polypropylene, polyethylene, polyurethane and polyester or any combination thereof. The polymer may be selected from the group consisting of: polypropylene, polyethylene, polyurethane and polyester or any combination thereof. The release liner(s) may comprise a polymer coated paper release liner. The protective liner may comprise a siliconized paper protective liner (also known as a silicone coated protective liner). The protective liner may comprise paper which is siliconized on one side. The protective liner may have a weight of between 60 gsm and 70 gsm, or preferably between 60 gsm and 65 gsm. The protective liner may comprise paper which is siliconized on one side and wherein the protective liner has a weight of between 60 gsm and 70 gsm, or preferably between 60 gsm and 65 gsm.

The protective liner may be transparent or translucent. This embodiment may be advantageous because it allows the user to visualise where the sealing strip is being applied before the protective liner is removed from the sealing strip.

The protective liner may comprise a handling tab formed from a portion of the protective liner which is not connected to the carrier layer. In some embodiments the protective liner may comprise two handling tabs each formed from a portion of the protective liner which is not connected to the carrier layer. In some embodiments the or each of the protective liner handling tabs may extend beyond one or each edge of the carrier layer, preferably at least one or both of the lateral edges.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the carrier layer is transparent or translucent.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the acrylic adhesive comprises no more than 20 wt.% solvent.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the adhesive layer is an essentially solvent free adhesive layer.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner is a siliconized paper release liner.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner has a weight or between 55 gsm and 70 gsm.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner is a siliconized paper release liner and the release liner has a weight or between 55 gsm and 70 gsm.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 30 gsm, and wherein the carrier layer has a weight of between 20 gsm and 35 gsm, and wherein the carrier layer is transparent or translucent.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 30 gsm, and wherein the carrier layer has a weight of between 20 gsm and 35 gsm, and wherein the acrylic adhesive comprises no more than 20 wt.% solvent.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 30 gsm, and wherein the carrier layer has a weight of between 20 gsm and 35 gsm, and wherein the adhesive layer is an essentially solvent free adhesive layer.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 30 gsm, and wherein the carrier layer has a weight of between 20 gsm and 35 gsm, and wherein the release liner is a siliconized paper release liner.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 30 gsm, and wherein the carrier layer has a weight of between 20 gsm and 35 gsm, and wherein the release liner has a weight or between 55 gsm and 70 gsm.

In preferred embodiments there is provided a sealing strip for a negative pressure wound dressing, the sealing strip comprising, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 30 gsm, and wherein the carrier layer has a weight of between 20 gsm and 35 gsm, and wherein the release liner is a siliconized paper release liner and the release liner has a weight or between 55 gsm and 70 gsm.

According to a second aspect of the invention there is provided a negative pressure wound therapy kit comprising a negative pressure wound therapy dressing and at least one sealing strip according to the first aspect of the invention.

The negative pressure wound therapy kit may comprise at least two sealing strips. The negative pressure wound therapy kit may comprise at least 4 sealing strips. The negative pressure wound therapy kit may comprise at least 6 sealing strips. The sealing strips may be detachably connected to each other at an edge of the sealing strip and by way of a perforated line.

The negative pressure wound therapy kit may comprise a negative pressure wound therapy dressing, a negative pressure source and at least one sealing strip. The negative pressure source may be a pump.

The negative pressure source may be removably connectable to the wound dressing. The negative pressure source may be removably connectable to the wound dressing by way of a connector. The connector may be a valve. The connector may be a one-way valve.

The negative pressure source may be removably connectable to the wound dressing by way of a conduit or tube. The negative pressure source may be removably connectable to the wound dressing by way of a connector and a conduit/tube.

In some preferred embodiments there is provided a negative pressure wound therapy kit comprising a negative pressure wound therapy dressing and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the carrier layer is transparent or translucent.

In some preferred embodiments there is provided a negative pressure wound therapy kit comprising a negative pressure wound therapy dressing and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the acrylic adhesive comprises no more than 20 wt.% solvent.

In some preferred embodiments there is provided a negative pressure wound therapy kit comprising a negative pressure wound therapy dressing and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the adhesive layer is an essentially solvent free adhesive layer.

In some preferred embodiments there is provided a negative pressure wound therapy kit comprising a negative pressure wound therapy dressing and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner is a siliconized paper release liner.

In some preferred embodiments there is provided a negative pressure wound therapy kit comprising a negative pressure wound therapy dressing and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner has a weight or between 55 gsm and 70 gsm.

In some preferred embodiments there is provided a negative pressure wound therapy kit comprising a negative pressure wound therapy dressing and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner is a siliconized paper release liner and the release liner has a weight or between 55 gsm and 70 gsm.

According to a third aspect of the invention there is provided a negative pressure wound therapy apparatus comprising a negative pressure wound therapy dressing, a negative pressure source and at least one sealing strip according to the first aspect of the invention, and wherein the negative pressure source is removably connectable to the wound dressing.

The negative pressure source may be removably connected to the wound dressing by way of a connector. The negative pressure source may be removably connected to the wound dressing by way of a conduit or tube.

In some preferred embodiments there is provided a negative pressure wound therapy apparatus comprising a negative pressure wound therapy dressing, a negative pressure source and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the carrier layer is transparent or translucent, and wherein the negative pressure source is removably connectable to the wound dressing.

In some preferred embodiments there is provided a negative pressure wound therapy apparatus comprising a negative pressure wound therapy dressing, a negative pressure source and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the acrylic adhesive comprises no more than 20 wt.% solvent, and wherein the negative pressure source is removably connectable to the wound dressing.

In some preferred embodiments there is provided a negative pressure wound therapy apparatus comprising a negative pressure wound therapy dressing, a negative pressure source and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the adhesive layer is an essentially solvent free adhesive layer, and wherein the negative pressure source is removably connectable to the wound dressing.

In some preferred embodiments there is provided a negative pressure wound therapy apparatus comprising a negative pressure wound therapy dressing, a negative pressure source and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner is a siliconized paper release liner, and wherein the negative pressure source is removably connectable to the wound dressing.

In some preferred embodiments there is provided a negative pressure wound therapy apparatus comprising a negative pressure wound therapy dressing, a negative pressure source and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner has a weight or between 55 gsm and 70 gsm, and wherein the negative pressure source is removably connectable to the wound dressing.

In some preferred embodiments there is provided a negative pressure wound therapy apparatus comprising a negative pressure wound therapy dressing, a negative pressure source and at least one sealing strip wherein the sealing strip comprises, in order: a carrier layer having opposite front and rear surfaces; an adhesive layer covering at least a portion of the front surface of the carrier layer and wherein the adhesive layer comprises an acrylic adhesive; and at least one release liner detachably connected to the adhesive layer; wherein the adhesive layer has a weight of no more than 33 gsm, and wherein the carrier layer has a weight of between 20 gsm and 35 gsm, and wherein the release liner is a siliconized paper release liner and the release liner has a weight or between 55 gsm and 70 gsm, and wherein the negative pressure source is removably connectable to the wound dressing.

### Detailed Description of the Invention

In order that the invention may be more clearly understood, embodiments thereof will now be described, by way of example only, with reference to the accompanying drawings, of which:
- Figure 1: illustrates a side cross sectional view of a first embodiment of a sealing strip of the first aspect of the invention.
- Figure 2: illustrates a bottom view of the first embodiment of the sealing strip of figure 1.
- Figure 3: is a graph of the average moisture vapour transfer rate of the first embodiment of the sealing strip of the invention and competitor sealing strips A and B.
- Figure 4: illustrates a side cross sectional view of a second embodiment of a sealing strip of the first aspect of the invention.

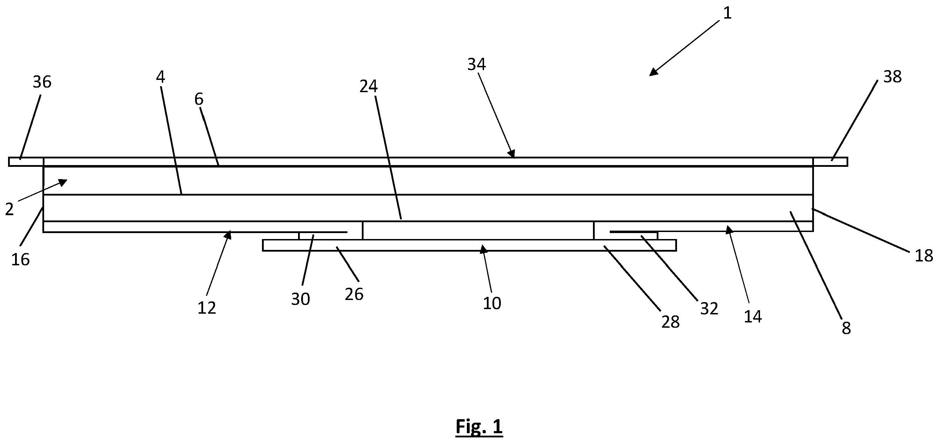

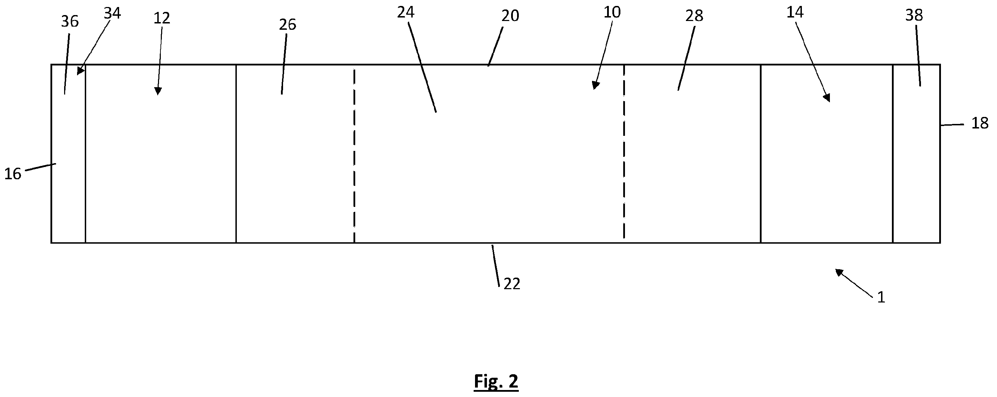

A first embodiment of a sealing strip (1) of the first aspect of the invention is illustrated in figure 1 (side cross-sectional view) and figure 2 (bottom view). The sealing strip (1) comprises a carrier layer (2) having opposite front (4) and rear surfaces (6); an adhesive layer (8) covering the front surface (4) and wherein the adhesive layer (8) comprises an acrylic adhesive; and three release liners; a first release liner (10), a second release liner (12) and a third release liner (14), each detachably connected to the adhesive layer (8); wherein the adhesive layer (8) has a weight of 25 gsm, and wherein the carrier layer (2) has a weight of 28 gsm. In other embodiments the adhesive layer (8) has a weight of no more than 30 gsm, 29 gsm, 27 gsm or no more than 26 gsm, and the carrier layer (2) has a weight of between 25 gsm and 35 gsm.

The acrylic adhesive is an essentially solvent free acrylic adhesive. The carrier layer (2) is a polyurethane material. The carrier layer (2) is flexible and translucent.

The sealing strip (1) is a rectangular shape comprising two opposing lateral short edges (16, 18) which are connected by two opposing long edges (20, 22). The sealing strip (1) comprises a central portion (24). The central portion (24) of the sealing strip (1) is illustrated in figure 2 by the dashed lines.

A central portion of the first release liner (10) is detachably connected to the adhesive layer (8) located on the central portion (24) of the sealing strip (1). A first portion of the first release liner (10) which is not detachably connected to the adhesive layer (8) comprises a handling tab (26) and a second portion of the first release liner (10) which is not detachably connected to the adhesive layer (8) comprises a second handling tab (28). The handling tabs (26, 28) are located at opposite ends of the first release liner (10).

The second release liner (12) extends from the central portion (24) of the sealing strip (1) to the first short edge (16) of the sealing strip (1). A portion of the second release liner (12) located towards the central portion (24) of the sealing strip (1) is not detachably connected to the adhesive layer (8) and comprises a handling tab (30).

The third release liner (14) extends from the central portion (24) of the sealing strip (1) to the second short edge (18) of the sealing strip (1). A portion of the third release liner (14) located towards the central portion (24) of the sealing strip (1) is not detachably connected to the adhesive layer (8) and comprises a handling tab (32).

The release liners (10, 12, 14) are siliconized paper wherein the weight of the siliconized paper is 63 gsm. In other embodiments the weight of the release liners (10, 12, 14) is between 50 gsm and 80 gsm or between 55 gsm and 70 gsm.

The sealing strip (1) further comprises a protective liner (34) detachably connected to the rear surface of the carrier layer (6). The protective liner (34) is detachably connected to the entire rear surface of the carrier layer (6). The protective liner (34) comprises a first handling tab (36) which extends beyond the first short edge (16) of the sealing strip (1) and a second handling tab (38) which extends beyond the second short edge (18) of the sealing strip (1).

A first embodiment of the negative pressure wound dressing kit according to the second aspect of the invention is provided wherein the negative pressure wound dressing kit comprises: a negative pressure wound dressing comprising a connector wherein the connector is detachably connected to a negative pressure source; and at least two, preferably six sealing strips of the first embodiment of the sealing strip (1) of the invention.

A first embodiment of the negative pressure wound therapy apparatus according to the third aspect of the invention is provided wherein the negative pressure wound therapy apparatus comprises a negative pressure wound therapy dressing, a negative pressure source and at least one sealing strip (1) of the first embodiment of the sealing strip of the invention, and wherein the negative pressure source is removably connectable to the wound dressing.

In use the negative pressure wound dressing is applied to a patient's wound and pressed firmly onto the surrounding skin. A sealing strip (1) is then applied to each edge of the wound dressing to ensure that a tight seal is formed so allow for a negative pressure to be applied without air leakage around the wound dressing.
In use, the sealing strip (1) is applied according to the following method:
1) The first release liner (10) is removed from the sealing strip (1) by way of the first handling tab (26) or by way of the second handling tab (28) to reveal the central portion of the adhesive layer (24);
2) The central portion of the adhesive layer (24) is applied over the edge of the wound dressing and pressed firmly;
3) The second release liner (12) is removed by way of the handling tab (30) and the exposed portion of the adhesive layer is then pressed firmly onto the skin;
4) The third release liner (14) is removed by way of the handling tab (32) and the exposed portion of the adhesive layer is then pressed firmly onto the skin; and
5) The protective layer (34) is removed from the rear surface of the sealing strip (6).

Alternatively in use, the sealing strip (1) is cut in half and one half is applied according to the following method;
1) The first release liner (10) is removed from the sealing strip (1) by way of the first handling tab (26) to reveal a first exposed portion of the adhesive layer;
2) The first exposed portion of the adhesive layer is applied over the edge of the wound dressing and pressed firmly;
3) The second release liner (12) is removed by way of the handling tab (30) and a second exposed portion of the adhesive layer is then pressed firmly onto the skin;
4) The protective layer (34) is removed from the rear surface of the sealing strip (6).

The other half may then be applied in the same manner.

The sealing strip (1) comprising the 25 gsm acrylic adhesive layer (8) and the 28 gsm polyurethane carrier layer (2) is advantageous because the sealing strip (1) has strong adhesive properties and a good moisture vapour transfer rate whilst still ensuring that it can be smoothly applied to the skin. Together, these features result in a sealing strip which results in longer wear time and is easier to apply. The acrylic adhesive layer (8) also results in less painful removal of the sealing strip (1).

The average thickness of the adhesive layer (8) and the carrier layer (2) of the inventive sealing strip (1) compared to 3 sealing strips of the prior art (A, B and C) are shown in table 1.

**Table 1:**

| **Sealing Strip** | **Average thickness (adhesive layer and carrier layer), /mm** |
|---|---|
| Inventive sealing strip (1) | 0.0507 |
| Known sealing strip A | 0.0590 |
| Known sealing strip B | 0.0683 |
| Known sealing strip C | 0.0653 |

Known sealing strip A comprises an acrylic medical adhesive on a clear polyurethane carrier. Known sealing strip B comprises a 40 gsm acrylic medical adhesive on a clear polyurethane carrier. Known sealing strip C comprises a 38 µm acrylic medical adhesive on a clear polyurethane carrier.

The adhesive layer (8) and carrier layer (2) thickness of the inventive sealing strip (1) is thinner than the adhesive and carrier layer thickness of each of the sealing strips of the prior art (A, B and C). Data was generated demonstrating the performance of the sealing strip (1) using the first embodiment of the negative pressure wound therapy apparatus on 24 healthy patients 7 days after application. Table 2 shows the performance of the inventive sealing strip (1) and 3 sealing strips of the prior art (A, B and C). The data shows the mean percentage of lifting over the 7 days, the pain on removal (mean pain score out of 10, wherein 0 is no pain and 10 is the worst pain imaginable) and the percentage of participants who report moderate to excessive force required to remove the sealing strip.

**Table 2:**

| **Sealing Strip** | **Mean percentage of lifting** | **Pain on removal (mean pain score)** | **Percentage of patients reporting force required to remove** |
|---|---|---|---|
| Inventive sealing strip (1) | 35 % | 0.74 | 38 % |
| Known sealing strip A | 19 % | 2.52 | 75 % |
| Known sealing strip B | 72 % | 0.27 | 8 % |
| Known sealing strip C | 80 % | 0.50 | 8 % |

The inventive sealing strip (1) showed a low percentage of lifting and a low pain on removal score which is advantageous for users because it results in a sealing strip which does not require frequent changing as it does not easily lift from the skin, but when it does require changing it does not hurt the patient. Known sealing strip A showed a lower percentage of lifting after 7 days but a high number of patients experienced higher pain on removal and stated that excess force was required to remove the sealing strip. Less lifting correlates with a longer wear time and therefore the inventive sealing strip (1) will not have to be changed as frequently or before required.

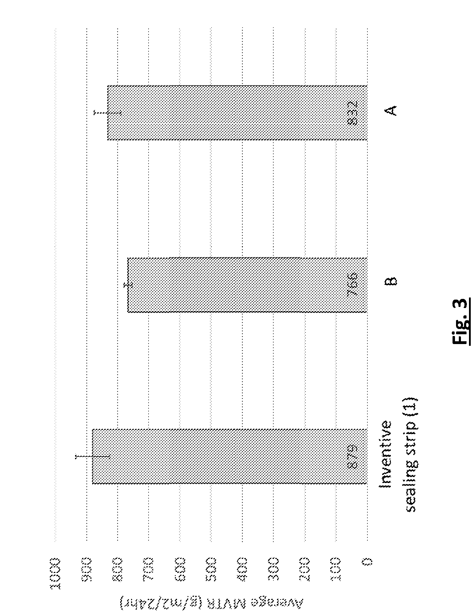

Figure 3 illustrates the average MVTR for the inventive sealing strip (1) compared to known sealing strips A and B. The inventive sealing strip (1) demonstrates superior MVTR which is advantageous because it ensures that moisture vapour is not trapped between the skin and the sealing strip (1). Trapped moisture vapour would negatively impact the adhesive properties of the sealing strip and therefore high MVTR improves adhesive properties and is correlated to increased wear time. Trapped moisture vapour would also cause maceration of the peri-wound skin which in turn would lead to pain or discomfort for the patient, and also may lead to an increased risk of infection; therefore, high MVTR is advantageous because it reduces the risk of maceration of the peri-wound skin.

The inventive sealing strip (1) comprising the 25 gsm acrylic adhesive layer (8) and the 28 gsm polyurethane carrier layer (2) is therefore advantageous because it results in a sealing strip with a lower amount of lifting, reduced pain on removal and increased MVTR, all of which lead to a sealing strip with a longer wear time and less risk of peri-wound skin maceration due to less trapped moisture.

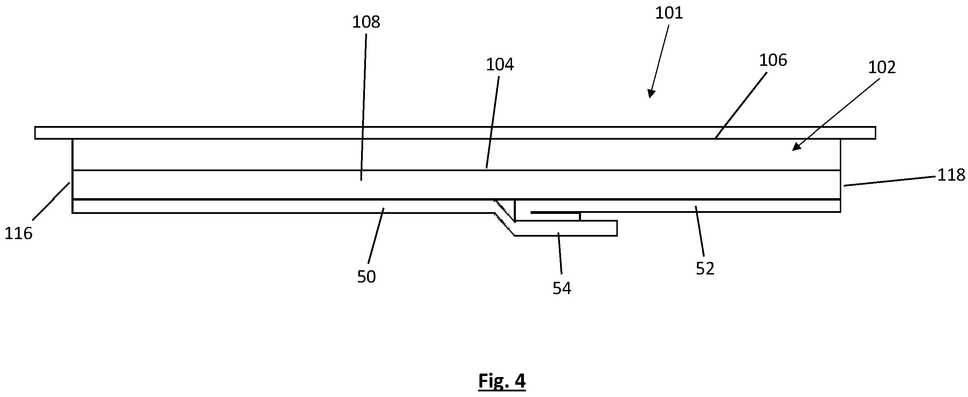

A second embodiment of a sealing strip (101) of the first aspect of the invention is illustrated in figure 4. The sealing strip (101) comprises a carrier layer (102) having opposite front (104) and rear surfaces (106); an adhesive layer (108) covering at least a portion of the front surface (104) and wherein the adhesive layer (108) comprises an acrylic adhesive; and two release liners (50, 52) detachably connected to the adhesive layer (108); wherein the adhesive layer (108) has a weight of 25 gsm, and wherein the carrier layer (102) has a weight of 28 gsm.

The second embodiment of the sealing strip (101) is essentially the same as the first embodiment of the sealing strip with the exception that there are two release liners (50, 52) instead of three. The first release liner (50) extends from the centre of the sealing strip to the first short edge (116) of the sealing strip. The second release liner (52) extends from the centre of the sealing strip to the first short edge (118) of the sealing strip. A portion (54) of the first release liner (50) overlaps with the second release liner (52) and the portion (54) forms a handling tab.

The second embodiment of the sealing strip (101) may be used in essentially the same way as the first embodiment of the sealing strip with the exception that the step comprising removing the third release liner is not required due to the absence of a third release liner.

A second embodiment of the negative pressure wound dressing kit according to the second aspect of the invention is provided wherein the negative pressure wound dressing kit comprises: a negative pressure wound dressing comprising a connector wherein the connector is detachably connected to a negative pressure source; and at least two, preferably six sealing strips of the second embodiment of the sealing strip of the invention (101).

A second embodiment of the negative pressure wound therapy apparatus according to the third aspect of the invention is provided wherein the negative pressure wound therapy apparatus comprises a negative pressure wound therapy dressing, a negative pressure source and at least one sealing strip of the second embodiment of the sealing strip of the invention (101), and wherein the negative pressure source is removably connectable to the wound dressing.

The above embodiments are described by way of example only. Many variations are possible without departing from the scope of the invention as defined in the appended claims.

## Claims

1. A sealing strip (1) for a negative pressure wound dressing, the sealing strip comprising, in order:
a carrier layer (2) having opposite front (4) and rear surfaces (6);
an adhesive layer (8) covering at least a portion of the front surface (4) of the carrier layer (2) and wherein the adhesive layer (8) comprises an acrylic adhesive; and
at least one release liner (10) detachably connected to the adhesive layer (8);
wherein the adhesive layer (8) has a weight of no more than 33 gsm, and
wherein the carrier layer (2) has a weight of between 20 gsm and 35 gsm.

2. A sealing strip (1) according to claim 1 wherein the adhesive layer (8) has a weight of no more than 30 gsm, or no more than 25 gsm.

3. A sealing strip (1) according to claim 1 or claim 2 wherein the carrier layer (2) comprises a polymer material, preferably wherein the carrier layer (2) comprises polyurethane.

4. A sealing strip (1) according to any preceding claim wherein the carrier layer (2) is transparent or translucent.

5. A sealing strip (1) according to any preceding claim wherein the adhesive layer (8) is an essentially solvent free adhesive layer.

6. A sealing strip (1) according to any preceding claim wherein the adhesive comprises no more than 20 wt.% solvent.

7. A sealing strip (1) according to any preceding claim wherein the adhesive layer (8) covers the entire front surface (4) of the carrier layer (2).

8. A sealing strip (1) according to any preceding claim wherein at least one, release liner (10) is detachably adhered to the adhesive layer, preferably wherein the or each release liner (10) is a paper release liner, and more preferably wherein the or each release liner (10) is a siliconized paper release liner.

9. A sealing strip (1) according to any preceding claim wherein the or each release liner (10) has a weight of between 60 gsm and 70 gsm.

10. A sealing strip (1) according to any preceding claim wherein a portion of the or each release liner(s) (10) comprises a handling tab (26) formed from a portion of the release liner which is not connected to the adhesive layer (8).

11. A sealing strip (1) according to any preceding claim, wherein the adhesive layer (8) has a weight of no more than 29 gsm, and wherein the carrier layer (2) has a weight of between 25 gsm and 30 gsm.

12. A negative pressure wound therapy kit comprising a negative pressure wound therapy dressing and at least one sealing strip (1) according to any one of claims 1 to 11.

13. A negative pressure wound therapy kit according to claim 12 wherein the negative pressure wound therapy kit comprises at least 2 sealing strips (1), preferably wherein the negative pressure wound therapy kit comprises at least 4 sealing strips (1) or at least 6 sealing strips (1).

14. A negative pressure wound therapy apparatus comprising a negative pressure wound therapy dressing, a negative pressure source and at least one sealing strip (1) according to any one of claims 1 to 11 and wherein the negative pressure source is removably connectable to the wound dressing.

15. A negative pressure wound therapy apparatus according to claim 14 wherein the negative pressure source is removably connected to the wound dressing by way of a connector; and/or wherein the negative pressure source is removably connected to the wound dressing by way of a conduit or tube.

## Patentansprüche

1. Dichtband (1) für einen Unterdruck-Wundverband, wobei das Dichtband in der folgenden Reihenfolge umfasst:
eine Trägerschicht (2), aufweisend einander gegenüberliegende Vorder- (4) und Rückoberflächen (6);
eine Klebstoffschicht (8), bedeckend mindestens einen Abschnitt der Vorderoberfläche (4) der Trägerschicht (2) und wobei die Klebstoffschicht (8) einen Acrylklebstoff umfasst; und
mindestens eine Trennlage (10), lösbar mit der Klebstoffschicht (8) verbunden;
wobei die Klebstoffschicht (8) ein Gewicht von höchstens 33 g/m² aufweist, und
wobei die Trägerschicht (2) ein Gewicht zwischen 20 g/m² und 35 g/m² aufweist.

2. Dichtband (1) gemäß Anspruch 1, wobei die Klebstoffschicht (8) ein Gewicht von höchstens 30 g/m², oder höchstens 25 g/m² aufweist.

3. Dichtband (1) gemäß Anspruch 1 oder Anspruch 2, wobei die Trägerschicht (2) ein Polymermaterial umfasst, bevorzugt wobei die Trägerschicht (2) Polyurethan umfasst.

4. Dichtband (1) gemäß einem der vorhergehenden Ansprüche, wobei die Trägerschicht (2) transparent oder transluzent ist.

5. Dichtband (1) gemäß einem der vorhergehenden Ansprüche, wobei die Klebstoffschicht (8) eine im Wesentlichen lösungsmittelfreie Klebstoffschicht ist.

6. Dichtband (1) gemäß einem der vorhergehenden Ansprüche, wobei der Klebstoff höchstens 20 Gew.-% Lösungsmittel umfasst.

7. Dichtband (1) gemäß einem der vorhergehenden Ansprüche, wobei die Klebstoffschicht (8) die gesamte Vorderoberfläche (4) der Trägerschicht (2) bedeckt.

8. Dichtband (1) gemäß einem der vorhergehenden Ansprüche, wobei mindestens eine Trennlage (10) lösbar an die Klebstoffschicht (8) geklebt ist, bevorzugt wobei die oder jede Trennlage (10) eine Papiertrennlage ist, und besonders bevorzugt wobei die oder jede Trennlage (10) eine silikonisierte Papiertrennlage ist.

9. Dichtband (1) gemäß einem der vorhergehenden Ansprüche, wobei die oder jede Trennlage (10) ein Gewicht zwischen 60 g/m² und 70 g/m² aufweist.

10. Dichtband (1) gemäß einem der vorhergehenden Ansprüche, wobei ein Abschnitt der oder jeder Trennlage (10) eine Grifflasche (26) umfasst, ausgebildet aus einem Abschnitt der Trennlage, der nicht mit der Klebstoffschicht (8) verbunden ist.

11. Dichtband (1) gemäß einem der vorhergehenden Ansprüche, wobei die Klebstoffschicht (8) ein Gewicht von höchstens 29 g/m² aufweist und wobei die Trägerschicht (2) ein Gewicht zwischen 25 g/m² und 30 g/m² aufweist.

12. Unterdruck-Wundtherapie-Kit, umfassend einen Unterdruck-Wundtherapieverband und mindestens ein Dichtband (1) gemäß einem der Ansprüche 1 bis 11.

13. Unterdruck-Wundtherapie-Kit gemäß Anspruch 12, wobei das Unterdruck-Wundtherapie-Kit mindestens zwei Dichtbänder (1) umfasst, bevorzugt wobei das Unterdruck-Wundtherapie-Kit mindestens vier Dichtbänder (1) oder mindestens sechs Dichtbänder (1) umfasst.

14. Unterdruck-Wundtherapie-Vorrichtung, umfassend einen Unterdruck-Wundtherapieverband, eine Unterdruckquelle und mindestens ein Dichtband (1) gemäß einem der Ansprüche 1 bis 11, wobei die Unterdruckquelle lösbar mit dem Wundverband verbindbar ist.

15. Unterdruck-Wundtherapie-Vorrichtung gemäß Anspruch 14, wobei die Unterdruckquelle mittels eines Verbinders lösbar mit dem Wundverband verbunden ist; und/oder wobei die Unterdruckquelle mittels einer Leitung oder eines Schlauchs lösbar mit dem Wundverband verbunden ist.

## Revendications

1. Une bande (1) d'étanchéité pour un pansement à dépression pour une plaie, la bande d'étanchéité comprenant, dans l'ordre :
une couche (2) de support ayant des surfaces opposées avant (4) et arrière (6) ;
une couche (8) adhésive recouvrant au moins une partie de la surface (4) avant de la couche (2) de support et dans laquelle la couche (8) adhésive comprend un adhésif acrylique ; et
au moins une doublure (10) antiadhésive reliée de manière détachable à la couche (8) adhésive ;
dans laquelle la couche (8) adhésive a un poids de pas plus de 33 gm², et
dans laquelle la couche (2) de support a un poids compris entre 20 gm² et 35 gm².

2. Une bande (1) d'étanchéité suivant la revendication 1, dans laquelle la couche (8) adhésive a un poids de pas plus de 30 gm² ou pas plus de 25 gm².

3. Une bande (1) d'étanchéité suivant la revendication 1 ou la revendication 2, dans laquelle la couche (2) de support comprend une matière polymère, dans laquelle, de préférence, la couche (2) de support comprend du polyuréthane.

4. Une bande (1) d'étanchéité suivant l'une quelconque des revendications précédentes, dans laquelle la couche (2) de support est transparente ou translucide.

5. Une bande (1) d'étanchéité suivant l'une quelconque des revendications précédentes, dans laquelle la couche (8) adhésive est une couche adhésive sensiblement exempte de solvant.

6. Une bande (1) d'étanchéité suivant l'une quelconque des revendications précédentes, dans laquelle la couche adhésive comprend pas plus de 20 % en poids de solvant.

7. Une bande (1) d'étanchéité suivant l'une quelconque des revendications précédentes, dans laquelle la couche (8) adhésive recouvre toute la surface (4) avant de la couche (2) de support.

8. Une bande (1) d'étanchéité suivant l'une quelconque des revendications précédentes, dans laquelle au moins une doublure (10) antiadhésive adhère de manière détachable à la couche adhésive, dans laquelle, de préférence, la ou chaque doublure (10) antiadhésive est une doublure antiadhésive en papier, et d'une manière encore plus préférée la ou chaque doublure (10) antiadhésive est une couche antiadhésive en papier siliconé.

9. Une bande (1) d'étanchéité suivant l'une quelconque des revendications précédentes, dans laquelle la ou chaque doublure (10) antiadhésive a un poids compris entre 60 gm² et 70 gm².

10. Une bande (1) d'étanchéité suivant l'une quelconque des revendications précédentes, dans laquelle une partie de ou de chaque doublure (10) antiadhésive comprend un onglet (26) de manipulation formé à partir d'une partie de la doublure antiadhésive, qui n'est pas reliée à la couche (8) adhésive.

11. Une bande (1) d'étanchéité suivant l'une quelconque des revendications précédentes, dans laquelle la couche (8) adhésive a un poids de pas plus de 29 gm², et dans laquelle la couche (2) de support a un poids compris entre 25 gm² et 30 gm².

12. Une trousse de thérapie à dépression pour une plaie comprenant un pansement de thérapie à dépression pour une plaie et au moins une bande (1) d'étanchéité suivant l'une quelconque des revendications 1 à 11.

13. Une trousse de thérapie à dépression pour une plaie de la revendication 12, dans laquelle la trousse de thérapie à dépression pour une plaie comprend au moins 2 bandes (1) d'étanchéité, dans laquelle, de préférence, la trousse de thérapie à dépression pour une plaie comprend au moins 4 bandes (1) d'étanchéité ou au moins 6 bandes (1) d'étanchéité.

14. Un dispositif de thérapie à dépression pour une plaie comprenant un pansement de thérapie à dépression pour une plaie, une source de dépression et au moins une bande (1) d'étanchéité suivant l'une des revendications 1 à 11, et dans lequel la source de dépression peut communiquer de manière amovible avec le pansement pour une plaie.

15. Un dispositif de thérapie à dépression pour une plaie suivant la revendication 14, dans lequel la source de dépression communique de manière amovible avec le pansement pour une plaie au moyen d'un connecteur ; et/ou dans lequel la source de dépression communique de manière amovible avec le pansement pour une plaie au moyen d'un conduit ou d'un tube.
